# EUROPEAN PATENT APPLICATION

(11) **EP 3 705 564 A1**
(43) Date of publication of application: **09.09.2020**
(21) Application number: 19020108.7
(22) Date of filing: 06.03.2019
(51) Int. Cl.: C12M 1/22, C12M 1/00, C12M 1/34

(54) **METHODS AND APPARATUS TO PROVIDE ENVIRONMENTAL CONTROL IN BIOLOGICAL SAMPLES CONTAINED IN CELL-CULTURE DISHES**

(71) Applicant: Cherry Biotech SAS, 35000 Rennes (FR)
(72) Inventor: Fassini, Dario, 14000 Caen (FR); Homs Corbera, Antoni, 17220 Sant Feliu de Guixols (ES)

(57) **Abstract**

The invention describes a device and method to obtain a controlled medium perfusion and drug injection/removal into standard cell culture dishes (Petri dishes). The device, once assembled to standard Petri dish substrates, allows continuous and discontinuous perfusion of medium and is suitable for short- or long-term culture. The specific architecture of the device can significantly reduce shear stress effects on cells also at relatively high flow rate. The device is hermetically sealed from the external environment, providing sterile condition outside dedicated culture cabinets, allowing to culture cells outside sterile environments, and can optionally be thermalized. Smooth and controlled drug injection/removal is possible. The device and methods here described are fully compatible with several microscopy techniques and allow to live imaging the sample maintaining the cells in a fully physiological environment.

## Description

### Technical Field

The invention concerns a device and associated methods providing short- and long-term *in vitro* culture of different biological materials. Particularly, but not exclusively, the device is suitable for the evaluation of long-term effect of therapeutic compounds, such as drugs, on biological materials. The sample can be perfused with fresh medium and the drug can be injected and removed while the sample is properly thermalized and monitored with suitable optical devices. The modularity of the device can allow interconnecting different biological materials/models to simulate different physiological processes, for example those related to long distances signaling.

### Background

Cell culture dishes, also known as Petri dishes honoring the bacteriologist Julius Richard Petri, is one of the most common enclosing formats, or standards, in which cells or biological tissues are cultured in a controlled static media. These polymeric or glass wares are used since decades in biological and pharmaceutical laboratories making them a standard accounting for many laboratory devices and protocols optimized for them.

Long term *in vitro* culture of biological materials requires strict control of the physical/chemical environment in order to maintain viability of cells. Whereas the biological mass grow along time nutrients are consumed and metabolic wastes accumulate [Lane et al, 2005, Zander et al, 2006]. This phenomenon is currently mitigated by periodically refreshing the culturing media. Nevertheless, this process may expose biological sample (i.e. embryos, mammalian cells, human tissues) to risks that include: potential for viability loss and/or introduction of contaminants; cellular stress during the medium renewal or drug injection, temporary temperature changes as well as pH fluctuations [Reed et al, 2009]. While most of the patents related to perfusion devices for cells/tissue culture are based on tailored polymeric devices, to the best of our knowledge, these methods and devices are not compatible with standard cell culture dishes, also referred as Petri dishes, when isolation and protection of the cellular environment from the external environment must be guaranteed.

Furthermore, existing devices permit only limited shear stress control which is also limiting the potential applications of such devices. Shear stress is a relevant factor known to alter the viability and physiology of cells. As an example, while some stem cells require shear stress to properly differentiate into specific phenotypes [Wolfe et al, 2013] uncontrolled shear stresses have a detrimental effect on other specific cells phenotypes [Liu et al, 2013]. While the shear stress induced by medium renewal of semi static cultures is generally not taken into consideration, the shear stress induced by dynamic culturing condition (i.e. perfused cells culture) must be consider in order to provide a suitable environment for the biological samples.

Temperature is another critical physical parameter that must be controlled to provide suitable environment for cells. In order to control temperature most of perfusing devices need to be placed into dedicated cells incubators or dedicated housing. US005595909A device must be placed inside a special incubator (AcuSyst, Cellex Biosciences Inc) while most of other devices are generally placed inside standard cell culture incubators to provide the right temperature (see for instance US20120135452A1 or US20050032208A1). This limits their operation and prevents the use of sophisticated imaging techniques to monitor the biological samples.

Another approach to control temperature is described in US20080032380A1 where the temperature of the culture is controlled by a dedicated but undescribed housing. In this case, an external Peltier module in contact with the device provides the suitable temperature; a solution also used in the CellASIC® ONIX2 Microfluidic System (Merk Millipore). An alternative method to control temperature in cultured cells relies in the usage of embedded electrodes near the culture chamber instead of a Peltier and by controlling it through a feedback loop based on a thermistor [Petronis et al, 2006].

Fluidic systems have been also used to control the temperature of the cell environment in cultures as it is done in the CherryTEMP (Cherry Biotech SAS) system which is based on FR2959678A1. However, these methods and apparats doesn't accomplish the simultaneous and precise control of perfused media and temperature of the cultured cells or tissues in their enclosing environment on the Petri dish standard.

The inexistence of a perfusion device based on standard cell culturing dishes, able to produce a controlled laminar flow, fine tune the physical/chemical parameters of cell and/or tissue cultures, and thus allowing to set the proper and cells-specific physiological environment is currently a challenge for life sciences, compound testing and stem cells differentiation studies.

### Summary of Invention

Culturing cells monolayers, cells in the form of tridimensional constructs or tissue biopsies in standard plastic or glassware like cell-culture dishes, also known as Petri dishes or Petri plates, is a common practice in all biological labs. In the last decades the importance of having a controlled environment and cells/organs/tissue interplay has been highlighted by several publications. It is a recurrent challenge to be able to perform biological experiments where the environment composition is permanently controlled and isolated from undesirable external environmental perturbations when using the standard cell culture plates. Most of current experimentation using standard cell culture plates rely on periodically replacing part of the cell culture media by automated or manual pipetting procedures which, besides limiting the biological culture viability to a few days, it exposes the biological samples to external and not fully controlled stresses and variabilities as well as potential contaminations.

The invention herein provides a solution to manipulate the physicochemical parameters inside standard cell-culture dishes using perfusion (i.e. altering temperature; shear stress; injecting a drug or a chemical compound; simulating hypoxia or normoxia) thus allowing to manipulate relevant biological processes while preventing external contaminations and environmental disturbs but allowing imaging samples under a microscope. The device and methods described in this invention provide a controlled medium perfusion and drug injection system for cell-culture dishes. Once assembled to standard cell-culture dish substrates, this invention allows continuous and/or discontinuous perfusion of medium and is suitable for short- or long-term culture. Its specific architecture can significantly control shear stress effects on cells while reducing it at relatively high flow rates of medium renewal. The device is fully sealed from the external environment, can be thermalized and provides sterile condition outside dedicated cabinet allowing to culture cells outside sterile environments. Controlled drug injection/removal is also possible.

Furthermore, this invention can be easily used to connect several cell culture dishes with their isolated controlled environments which is another advantage of the invention for experiments where multiple biological samples should be connected to study their biochemical interactions.

### Brief Description of the Drawings

**Fig. 1** is a bottom-up perspective view of the device, including the cell-culture dish base where it will be assembled, in accordance with one of the preferred embodiments of the present invention.
**Fig. 2** is a Top-down perspective view of the device, including the cell-culture dish base where it will be assembled, in accordance with one of the preferred embodiments of the present invention as shown in Fig. 1 and for the case of integrating only one inlet and one outlet entries for the fluids.
**Fig. 3** is an exploded perspective view of the device, including the cell-culture dish base where it will be assembled, in accordance with the preferred embodiment of the present invention as shown in Fig. 1 and Fig. 2.
**Fig. 4** is an exploded perspective view of the device, including the cell-culture dish base where it will be assembled, in accordance with the preferred embodiment of the present invention as shown in

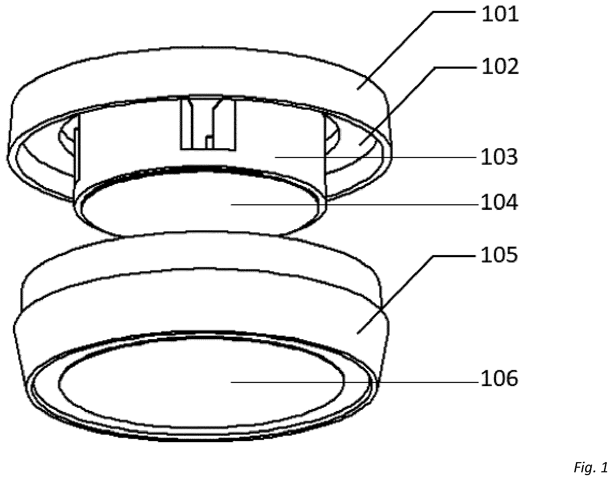

Fig. 1 and for the case of integrating only two inlet and two outlet entries for the fluids as well as a microfluidic thermalization chamber non fluidically communicated with the biological sample.

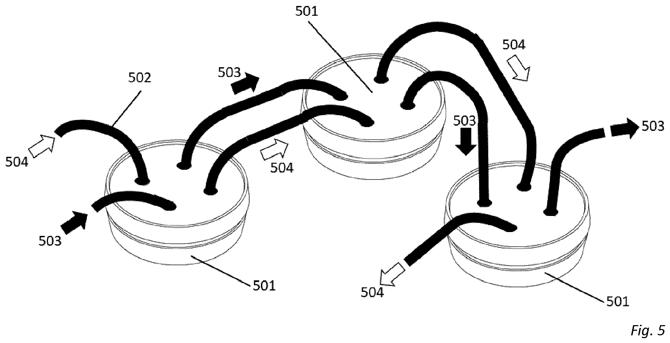

**Fig. 5** is an illustration on how different embodiments of the invention can be combined to produce more complex biological and compound testing experiments.

### Detailed Description of the Drawings (or Embodiments)

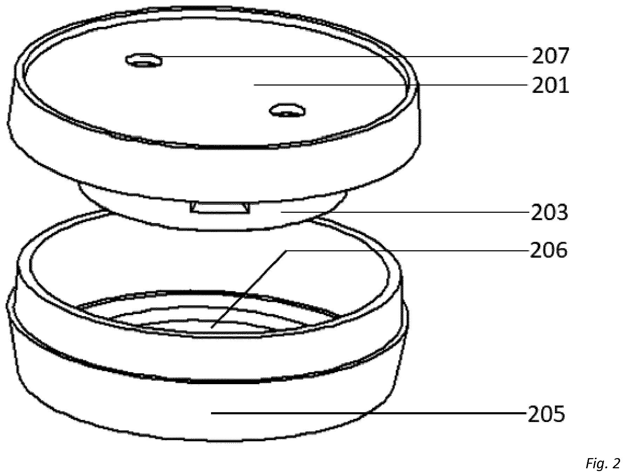

Referring to the drawings in Fig. 1 and Fig. 2 , a method and device to provide environmental control in biological samples contained in a cell-culture dish in accordance to one of the preferred embodiments of the present invention comprises an hermetically sealing cap or lid (101 and 201) with embedded optically transparent parts with at least two orifices (207) connecting the exterior and the interior environments of a cell-culture dish (105 and 205) once hermetically sealed by the cap or lid (101 and 201), a flexible or elastic gasket (102) providing fluidic sealing between the cap or lid (101 and 201) and the cell-culture dish (105 and 205), a manifold (103 and 203) attached to or embedded into the cap or lid (101 and 201) providing fluidic connections and circuitry between the exterior and the interior environments of the cell-culture dish (105 and 205) once hermetically sealed by the cap or lid (101 and 201), a transparent flat surface (104) bonded or attached to the bottom of the manifold (103 and 203) providing the required sealing for the fluidic circuitry of the manifold (103 and 203), and a cell-culture dish or similar structure (105 and 205) containing an optically transparent part (106 and 206).

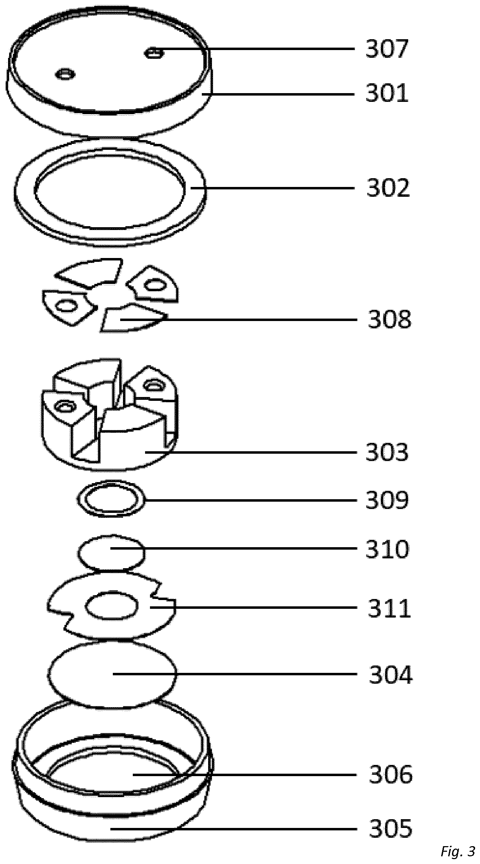

A more detailed exploded drawing of one of the preferred embodiments of the present invention is provided in Fig. 3 and comprises an hermetically sealing cap or lid (301) with embedded optically transparent parts with at least two orifices (307) connecting the exterior and the interior environments of a cell-culture dish (305) once hermetically sealed by the cap or lid (301), a flexible or elastic gasket (302) providing hermetic fluidic sealing between the cap or lid (301) and the cell-culture dish (305), a manifold (303) attached to the cap or lid (301) by means of double side tape adhesive material or an adhesive layer (308) providing a central optically transparent aperture and fluidic connections and circuitry between the exterior and the interior environments of the cell-culture dish (305) once hermetically sealed by the cap or lid (301), a transparent flat surface (310) bonded or attached to the bottom of the manifold (303)) by means of double side tape adhesive material or an adhesive layer (309) providing the required sealing for the fluidic circuitry (311) attached to the bottom of the manifold, a fluidic structure (311) providing connections between the exterior and the interior fluidic environments of the cell-culture dish (305) with a central space providing an optically transparent aperture that could be defined by patterning a double side adhesive tape or by directly engraving the manifold (403), an optically transparent flat surface (304) bonded or attached to the fluidic structure (311) providing the required sealing and selective openings for this fluidic circuitry (311) attached to the bottom of the manifold (303), and a cell-culture dish or similar structure (305) containing an optically transparent part (306).

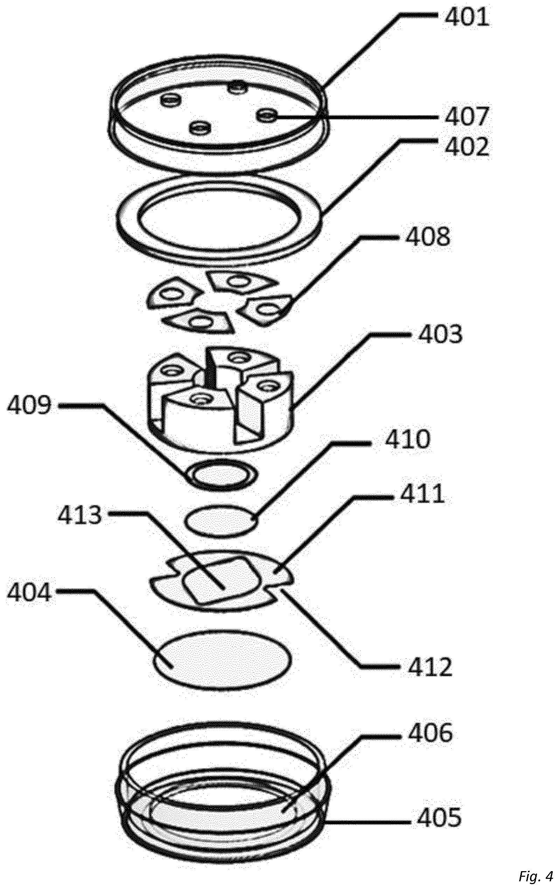

A more detailed exploded drawing of another preferred embodiment of the present invention, aimed to simultaneous provide perfusion of external fluids inside the sealed cell culture chamber and thermalization of the sealed cell culture chamber by using an externally temperature controlled thermalization fluid, is provided in Fig. 4 and comprises an hermetically sealing cap or lid (401) with embedded optically transparent parts with at least four orifices (407) connecting the exterior and the interior environments of a cell-culture dish (405) and providing the connection for the thermalized solution to the manifold (403) once hermetically sealed by the cap or lid (401), a flexible or elastic gasket (402) providing fluidic sealing between the cap or lid (401) and the cell-culture dish (405), a manifold (403) attached to the cap or lid (401) by means of double side tape adhesive material or an adhesive layer (408) providing a central optically transparent aperture and fluidic connections and circuitry between the exterior and the interior environments of the cell-culture dish (405) once hermetically sealed by the cap or lid (401), a transparent flat surface (410) bonded or attached to the bottom of the manifold (403) by means of double side tape adhesive material or an adhesive layer (409) providing the required sealing for the fluidic circuitry (411) attached to the bottom of the manifold, a fluidic structure (411) providing connections between the exterior and the interior fluidic environments (412) of the cell-culture dish (405) and providing connections for the isolated thermalizing fluidics (413) circulating the externally provided thermalized fluids in the manifold (403) that could be defined by patterning a double side adhesive tape or by directly engraving the manifold (403), an optically transparent flat surface (404) bonded or attached to the fluidic structure (411) providing the required sealing and selective openings for this fluidic circuitry (412) attached to the bottom of the manifold (403), and a cell-culture dish or similar structure (405) containing an optically transparent part (406).

One or more fluidic layers forming a sandwich of stacked 311 and 304 parts or 411 and 404 can be used to create different microfluidic patterns in the 3D plane.

A method based in connecting at least two of the preferred embodiments to emulate the interactions happening between biological structures present in nature and to accomplish better emulations of the biophysical and biochemical interactions for compounds testing is drawn in Fig. 5. The method could be used to replicate complex physiologies and to mimic multi organ interactions of crucial importance to better test therapeutic and other compounds *in vitro.* The method comprises two or more embodiments of the present invention (501), multiple fluidic connections (502) between the embodiments and from the embodiments (501) to the externally provided fluids (503 and 504), one or more defined fluidic routes (503) connecting several embodiments (501) for externally provided fluids reaching the enclosed biological samples, and alternatively one or more additional fluidic routes (504) connecting externally provided thermalizing fluids circulating through the manifolds of the embodiments (501) without directly contacting the enclosed biological samples.

Although the invention has been explained in relation to its preferred embodiment(s) as mentioned above, it is to be understood that many other possible modifications and variations can be made without departing from the scope of the present invention. It is, therefore, contemplated that the appended claim or claims will cover such modifications and variations that fall within the true scope of the invention.

### Industrial Applicability

The invention is applicable to the healthcare, pharma, life sciences and biotech industrial fields and more in particular as a tool for drug testing, drug screening, therapeutics development and for the evaluation of toxicological effects of compounds.

### Citation List

### Patent Literature

Hu W.-S. and Cerra F. B. Filter Device. 1997; US005595909A.
Kleis S. J., Geffert S. K. and Gonda S. R. Method and apparatus for a miniature bioreactor system for long-term cell culture. 2008; US20080032380A1.
Oh S. K. W. and Chou A. B. H. Materials and methods to produce stem cells. 2005; US20050032208A1.
Shuler M. L. and Sung J. H. Microfluidic device for pharmacokinetic-pharmacodynamic study of drugs and uses thereof. 2012; US20120135452A1.
Yong C., Le Berre M., Velve Casquillas G. and Plecis A. Microfluidic chip, carrier system and implementation method for a spatially and fast controlled thermal regulation of a sample. 2010; FR2959678A1.

### Non Patent Literature

Lane M. and Gardner D.K. Understanding cellular disruptions during early development that perturb viability and fetal development. Reprod Fertil Dev. 2005; 17: 371 - 378.
Liu M., Song W., Li P., Huang Y., Gong X, Zhou G., Jia X., Zheng L. and Fan Y. Galanin Protects against Nerve Injury after Shear Stress in Primary Cultured Rat Cortical Neurons. PLoS ONE. 2013; 8(5), e63473.
Petronis S., Stangegaard M., Christensen C. B. and Dufva M. Transparent polymeric cell culture chip with integrated temperature control and uniform media perfusion. BioTechniques. 2006; 40(3), 368 - 376.
Reed M. L., Hamic A. and Caperton C. L. Continuous uninterrupted single medium culture without medium renewal versus sequential media culture: a sibling embryo study. Fertil Steril. 2009; 92(5), 1783 -1786.
Wolfe R. P. and Ahsan T. Shear stress during early embryonic stem cell differentiation promotes hematopoietic and endothelial phenotypes. Biotechnol Bioeng. 2013; 110(4), 1231 - 1242.
Zander D. L., Thompson J. G. and Lane M. Perturbations in mouse embryo development and viability caused by ammonium are more severe after exposure at the cleavage stages. Biol Reprod. 2006; 74: 288 - 294.

## Claims

1. A novel method and device that, once attached to standard cell culture plates, allows permanent control of the biological culture shear stress and medium composition while also permitting continuous microscopy-based imaging as well as isolation of contained biological culture environment from its surrounding environment. The method and device allow the selective manipulation of relevant biological processes and the study of compounds-induced changes in biological samples, such as cells, organoids, biopsies, organisms, embryos or tissue cultures, in hermetically closed and environmentally controlled cell culture plates. The device, comprises:
a lid, or cap, containing optically transparent parts and hermetically sealing the corresponding standard cell culture dish with at least two orifices, providing at least one inlet and at least one outlet, to externally supply and control fluids continuous or discontinuous injection, where fluids could be any gas or liquid which could be relevant for a biological experiment.
a manifold with a top part attached to the lid providing the guidance of the externally supplied fluids into the biological culture environment of the cell culture dish by a given set or orifices and microchannels without compromising the passing of light through some of the lid, or cap, optically transparent parts.

2. The method and device in accordance to claim 1, wherein:
at least one microfluidic circuitry layer and its corresponding sealing layer is embedded or attached to the bottom part of the manifold allowing the guidance of the fluids which are provided externally through the manifold into the biological culture environment of the cell culture dish.

3. The method and device in accordance to claim 1, wherein:
at least one stacked microfluidic circuitry layer and its corresponding sealing layer is embedded or attached to the bottom part of the manifold allowing the guidance of externally thermalized fluids which are provided through the manifold to control the temperature of the biological culture environment of the cell culture dish without affecting the chemical or biochemical composition of this said biological culture environment.

4. The method and device in accordance to claim 1, wherein:
a lid, or cap, or at least one tube fluidically connect two or more devices such as the one described in claim 1 permitting selective passage of part of the biological culture environment from one or more cell culture dish/es into one or more cell culture dish/es.
